# EUROPEAN PATENT SPECIFICATION

(11) **EP 1 333 840 B1**
(45) Date of publication and mention of the grant of the patent: **07.03.2007**
(21) Application number: 01983694.9
(22) Date of filing: 16.11.2001
(51) Int. Cl.: A61K 31/711, A61K 39/04, A61P 31/04

(54) **M. TUBERCULOSIS CHAPERONIN 60.1 AND USES THEREOF**
M. TUBERCULOSIS CHAPERONIN 60.1 UND SEINE VERWENDUNGEN
M.TUBERCULOSIS CHAPERONIN 60.1 ET SES UTILISATIONS

(30) Priority: 17.11.2000 GB 0028122
(43) Date of publication of application: 13.08.2003
(60) Divisional application: 05077510.5
(73) Proprietor: Helperby Therapeutics Limited, Selby North Yorkshire YO8 4PW (GB)
(72) Inventor: COATES, Anthony R. M., c/o Dept. of Med. Microbio., Tooting, London SW17 (GB)
(74) Representative: Thomas, Philip John Duval
(86) International application number: PCT/GB2001/005069
(87) International publication number: WO 2002/040037

(56) References cited:
- WO-A-99/35270
- DATABASE SWALL [Online] EBI; 1 November 1997 (1997-11-01) "60 kDa chaperonin 1 of M. tuberculosis" Database accession no. Q59573 XP002203460

### SEQUENCE LISTING

<110> St George's Enterprises
   <120> Biological materials and uses thereof
<130> GEOT/P25182PC
<140> PCT/GB01/05069
   <141> 16 November 2001
<150> GB0028122.0
   <151> 17 November 2000
<160> 30
<170> SeqWin99
<210> 1
   <211> 8
   <212> PRT
   <213> Unknown
<220>
   <223> Polypeptide derived from various Mycobacterium species
<400> 1
   Met Ser Lys Leu Ile Glu Tyr Asp
   1 5
<210> 2
   <211> 8
   <212> PRT
   <213> Unknown
<220>
   <223> Polypeptide derived from various Mycobacterium species
<400> 2
   Ala Met Glu Val Gly Met Asp Lys
   1 5
<210> 3
   <211> 9
   <212> PRT
   <213> Unknown
<220>
   <223> Polypeptide derived from various Mycobacterium species
<400> 3
   Ala Lys Ala Phe Gly Gly Pro Thr Val
   1 5
<210> 4
   <211> 8
   <212> PRT
   <213> Unknown
<220>
   <223> Polypeptide derived from various Mycobacterium species
<400> 4
   Pro Phe Glu Asp Leu Gly Ala Gln
   1 5
<210> 5
   <211> 10
   <212> PRT
   <213> Unknown
<220>
   <223> Polypeptide derived from various Mycobacterium species
<400> 5
   Gln Ala Leu Ile Lys Gly Gly Leu Arg Leu
   1 5 10
<210> 6
   <211> 20
   <212> PRT
   <213> Unknown
<220>
   <223> Polypeptide derived from various Mycobacterium species
<400> 6
   Val Asn Pro Ile Ala Leu Gly Val Gly Ile Gly Lys Ala Ala Asp Ala
   1 5 10 15
   Val Ser Glu Ala
   20
<210> 7
   <211> 12
   <212> PRT
   <213> Unknown
<220>
   <223> Polypeptide derived from various Mycobacterium species
<400> 7
   Ala Ser Ala Thr Pro Val Ser Gly Lys Thr Gly Ile
   1 5 10
<210> 8
   <211> 12
   <212> PRT
   <213> Unknown
<220>
   <223> Polypeptide derived from various Mycobacterium species
<400> 8
   Ala Gln Val Ala Thr Val Ser Ser Arg Asp Glu Gln
   1 5 10
<210> 9
   <211> 16
   <212> PRT
   <213> Unknown
<220>
   <223> Polypeptide derived from various Mycobacterium species
<400> 9
   Val Gly Glu Ala Met Ser Lys Val Gly His Asp Gly Val Val Ser Val
   1               5                   10                  15
<210> 10
   <211> 22
   <212> PRT
   <213> Unknown
<220>
   <223> Polypeptide derived from various Mycobacterium species
<400> 10
   Ser Thr Leu Gly Thr Glu Leu Glu Phe Thr Glu Gly Ile Gly Phe Asp
   1               5                   10                  15
   Lys Gly Phe Leu Ser Ala
               20
<210> 11
   <211> 25
   <212> PRT
   <213> Unknown
<220>
   <223> Polypeptide derived from various Mycobacterium species
<400> 11
   Lys Gly Phe Leu Ser Ala Tyr Phe Val Thr Asp Phe Asp Asn Gln Gln
   1               5                   10                  15
   Ala Val Leu Glu Asp Ala Leu Ile Leu
               20                  25
<210> 12
   <211> 14
   <212> PRT
   <213> Unknown
<220>
   <223> Polypeptide derived from various Mycobacterium species
<400> 12
   Phe Asp Asn Gln Gln Ala Val Leu Glu Asp Ala Leu Ile Leu
   1               5                   10
<210> 13
   <211> 9
   <212> PRT
   <213> Unknown
<220>
   <223> Polypeptide derived from various Mycobacterium species
<400> 13
   His Gln Asp Lys Ile Ser Ser Leu Pro
   1               5
<210> 14
   <211> 8
   <212> PRT
   <213> Unknown
<220>
   <223> Polypeptide derived from various Mycobacterium species
<400> 14
   Ala Ile Arg Lys Thr Leu Lys Ala
   1               5
<210> 15
   <211> 18
   <212> PRT
   <213> Unknown
<220>
   <223> Polypeptide derived from various Mycobacterium species
<400> 15
   Gly Pro Tyr Phe Gly Asp Arg Arg Lys Ala Phe Leu Glu Asp Leu Ala
   1               5                   10                  15
   Val Val
<210> 16
   <211> 16
   <212> PRT
   <213> Unknown
<220>
   <223> Polypeptide derived from various Mycobacterium species
<400> 16
   Val Asn Pro Asp Ala Gly Met Val Leu Arg Glu Val Gly Leu Glu Val
   1               5                   10                  15
<210> 17
   <211> 12
   <212> PRT
   <213> Unknown
<220>
   <223> Polypeptide derived from various Mycobacterium species
<400> 17
   Leu Gly Ser Ala Arg Arg Val Val Val Ser Lys Asp
   1               5                   10
<210> 18
   <211> 16
   <212> PRT
   <213> Unknown
<220>
   <223> Polypeptide derived from various Mycobacterium species
<400> 18
   Asp Thr Val Ile Val Asp Gly Gly Gly Thr Ala Glu Ala Val Ala Asn
   1               5                   10                  15
<210> 19
   <211> 11
   <212> PRT
   <213> Unknown
<220>
   <223> Polypeptide derived from various Mycobacterium species
<400> 19
   Arg Ala Lys His Leu Arg Ala Glu Ile Asp Lys
   1               5                   10
<210> 20
   <211> 13
   <212> PRT
   <213> Unknown
<220>
   <223> Polypeptide derived from various Mycobacterium species
<400> 20
   Val Gly Ala Ala Thr Glu Thr Ala Leu Lys Glu Arg Lys
   1               5                   10
<210> 21
   <211> 9
   <212> PRT
   <213> Unknown
<220>
   <223> Polypeptide derived from various Mycobacterium species
<400> 21
   Glu Ser Val Glu Asp Ala Val Ala Ala
   1               5
<210> 22
   <211> 23
   <212> PRT
   <213> Unknown
<220>
   <223> Polypeptide derived from various Mycobacterium species
<400> 22
   Pro Gly Gly Gly Ala Ser Leu Ile His Gln Ala Arg Lys Ala Leu Thr
   1               5                   10                  15
   Glu Leu Arg Ala Ser Leu Thr
               20
<210> 23
   <211> 16
   <212> PRT
   <213> Unknown
<220>
   <223> Polypeptide derived from various Mycobacterium species
<400> 23
   Gly Pro Glu Val Leu Gly Val Asp Val Phe Ser Glu Ala Leu Ala Ala
   1               5                   10                  15
<210> 24
   <211> 14
   <212> PRT
   <213> Unknown
<220>
   <223> Polypeptide derived from various Mycobacterium species
<400> 24
   Pro Leu Phe Trp Ile Ala Ala Asn Ala Gly Leu Asp Gly Ser
   1               5                   10
<210> 25
   <211> 8
   <212> PRT
   <213> Unknown
<220>
   <223> Polypeptide derived from various Mycobacterium species
<400> 25
   Val Val Val Asn Lys Val Ser Glu
   1               5
<210> 26
   <211> 15
   <212> PRT
   <213> Unknown
<220>
   <223> Polypeptide derived from various Mycobacterium species
<400> 26
   Val Asn Thr Leu Ser Tyr Gly Asp Leu Ala Ala Asp Gly Val Ile
   1               5                   10                  15
<210> 27
   <211> 26
   <212> PRT
   <213> Unknown
<220>
   <223> Polypeptide derived from various Mycobacterium species
<400> 27
   Arg Ser Ala Val Leu Asn Ala Ser Ser Val Ala Arg Met Val Leu Thr
   1               5                   10                  15
   Thr Glu Thr Val Val Val Asp Lys Pro Ala
               20                  25
<210> 28
   <211> 13
   <212> PRT
   <213> Unknown
<220>
   <223> Polypeptide derived from various Mycobacterium species
<400> 28
   Lys Ala Glu Asp His Asp His His His Gly His Ala His
   1               5                   10
<210> 29
   <211> 1620
   <212> DNA
   <213> Mycobacterium tuberculosis
<400> 29
   atgagcaagc tgatcgaata cgacgaaacc gcgcgtcgcg ccatggaggt cggcatggac    60
   aagctggccg acaccgtgcg ggtgacgctg gggccgcgcg gccggcatgt ggtgctggcc   120
   aaggcgtttg gcggacccac ggttaccaac gacggcgtca cggtggcacg tgagatcgag   180
   ctggaagatc cgtttgaaga cttgggcgcc cagctggtga agtcggtggc caccaagacc   240
   aacgatgtgg ccggtgacgg caccaccacc gcaaccatct tggcgcaggc actgatcaag   300
   ggcggcctga ggctagtggc cgccggcgtc aacccgatcg cgctcggcgt gggaatcggc   360
   aaggccgccg acgcggtatc cgaggcgctg ctggcatcgg ccacgccggt gtccggcaag   420
   accggcatcg cgcaggtggc gacggtgtcc tcgcgcgacg agcagatcgg tgacctggtt   480
   ggcgaagcga tgagcaaggt cggccacgac ggcgtggtca gcgtcgaaga atcctcgacg   540
   ctgggcaccg agttggagtt caccgagggt atcggcttcg acaagggctt cttgtcggca   600
   tacttcgtta ccgacttcga taaccagcag gcggtgctcg aggacgcgtt gatcctgctg   660
   caccaagaca agatcagctc gcttcccgat ctgttgccat tgctggaaaa ggttgcagga   720
   acgggtaagc cactactgat cgtggctgaa gacgtggagg gcgaagcgtt ggcgacgctg   780
   gtcgtcaacg cgattcgcaa gacgttgaaa gcggtcgcgg tcaaggggcc gtacttcggt   840
   gaccgccgta aggcgttcct tgaggacctg gcggtggtga cgggtggcca ggtggtcaac   900
   cccgacgccg gcatggtgct gcgcgaggtg ggcttggagg tgctgggctc ggcccgacgc   960
   gtggtggtca gcaaggacga cacggtcatt gtcgacggcg gcggcaccgc agaagcggtg  1020
   gccaaccggg cgaagcactt gcgtgccgag atcgacaaga gcgattcgga ttgggatcgg  1080
   gaaaagcttg gcgagcggct ggccaaactg gccggcgggg ttgctgtcat caaggtgggt  1140
   gccgccaccg agaccgcact caaggagcgc aaggaaagcg tcgaggatgc ggtcgcggcc  1200
   gccaaggccg cggtcgagga gggcatcgtc cctggtgggg gagcctcgct catccaccag  1260
   gcccgcaagg cgctgaccga actgcgtgcg tcgctgaccg gtgacgaggt cctcggtgtc  1320
   gacgtgttct ccgaagccct tgccgcgccg ttgttctgga tcgccgccaa cgctggcttg  1380
   gacggctcgg tggtggtcaa caaggtcagc gagctacccg ccgggcatgg gctgaacgtg  1440
   aacaccctga gctatggtga cttggccgct gacggcgtca tcgacccggt caaggtgact  1500
   aggtcggcgg tgttgaacgc gtcatcggtt gcccggatgg tactcaccac cgagacggtc  1560
   gtggtcgaca agccggccaa ggcagaagat cacgaccatc accacgggca cgcgcactga  1620
<210> 30
   <211> 539
   <212> PRT
   <213> Mycobacterium tuberculosis
<400> 30
   Met Ser Lys Leu Ile Glu Tyr Asp Glu Thr Ala Arg Arg Ala Met Glu
   1               5                   10                  15
   Val Gly Met Asp Lys Leu Ala Asp Thr Val Arg Val Thr Leu Gly Pro
               20                  25                  30
   Arg Gly Arg His Val Val Leu Ala Lys Ala Phe Gly Gly Pro Thr Val
           35                  40                  45
   Thr Asn Asp Gly Val Thr Val Ala Arg Glu Ile Glu Leu Glu Asp Pro
       50                  55                  60
   Phe Glu Asp Leu Gly Ala Gln Leu Val Lys Ser Val Ala Thr Lys Thr
   65                  70                  75                  80
   Asn Asp Val Ala Gly Asp Gly Thr Thr Thr Ala Thr Ile Leu Ala Gln
                   85                  90                  95
   Ala Leu Ile Lys Gly Gly Leu Arg Leu Val Ala Ala Gly Val Asn Pro
               100                 105                 110
   Ile Ala Leu Gly Val Gly Ile Gly Lys Ala Ala Asp Ala Val Ser Glu
           115                 120                 125
   Ala Leu Leu Ala Ser Ala Thr Pro Val Ser Gly Lys Thr Gly Ile Ala
       130                 135                 140
   Gln Val Ala Thr Val Ser Ser Arg Asp Glu Gln Ile Gly Asp Leu Val
   145                 150                 155                 160
   Gly Glu Ala Met Ser Lys Val Gly His Asp Gly Val Val Ser Val Glu
                   165                 170                 175
   Glu Ser Ser Thr Leu Gly Thr Glu Leu Glu Phe Thr Glu Gly Ile Gly
               180                 185                 190
   Phe Asp Lys Gly Phe Leu Ser Ala Tyr Phe Val Thr Asp Phe Asp Asn
           195                 200                 205
   Gln Gln Ala Val Leu Glu Asp Ala Leu Ile Leu Leu His Gln Asp Lys
       210                 215                 220
   Ile Ser Ser Leu Pro Asp Leu Leu Pro Leu Leu Glu Lys Val Ala Gly
   225                 230                 235                 240
   Thr Gly Lys Pro Leu Leu Ile Val Ala Glu Asp Val Glu Gly Glu Ala
                   245                 250                 255
   Leu Ala Thr Leu Val Val Asn Ala Ile Arg Lys Thr Leu Lys Ala Val
               260                 265                 270
   Ala Val Lys Gly Pro Tyr Phe Gly Asp Arg Arg Lys Ala Phe Leu Glu
           275                 280                 285
   Asp Leu Ala Val Val Thr Gly Gly Gln Val Val Asn Pro Asp Ala Gly
       290                 295                 300
   Met Val Leu Arg Glu Val Gly Leu Glu Val Leu Gly Ser Ala Arg Arg
   305                 310                 315                 320
   Val Val Val Ser Lys Asp Asp Thr Val Ile Val Asp Gly Gly Gly Thr
                   325                 330                 335
   Ala Glu Ala Val Ala Asn Arg Ala Lys His Leu Arg Ala Glu Ile Asp
               340                 345                 350
   Lys Ser Asp Ser Asp Trp Asp Arg Glu Lys Leu Gly Glu Arg Leu Ala
           355                 360                 365
   Lys Leu Ala Gly Gly Val Ala Val Ile Lys Val Gly Ala Ala Thr Glu
       370                 375                 380
   Thr Ala Leu Lys Glu Arg Lys Glu Ser Val Glu Asp Ala Val Ala Ala
   385                 390                 395                 400
   Ala Lys Ala Ala Val Glu Glu Gly Ile Val Pro Gly Gly Gly Ala Ser
                   405                 410                 415
   Leu Ile His Gln Ala Arg Lys Ala Leu Thr Glu Leu Arg Ala Ser Leu
               420                 425                 430
   Thr Gly Asp Glu Val Leu Gly Val Asp Val Phe Ser Glu Ala Leu Ala
           435                 440                 445
   Ala Pro Leu Phe Trp Ile Ala Ala Asn Ala Gly Leu Asp Gly Ser Val
       450                 455                 460
   Val Val Asn Lys Val Ser Glu Leu Pro Ala Gly His Gly Leu Asn Val
   465                 470                 475                 480
   Asn Thr Leu Ser Tyr Gly Asp Leu Ala Ala Asp Gly Val Ile Asp Pro
                   485                 490                 495
   Val Lys Val Thr Arg Ser Ala Val Leu Asn Ala Ser Ser Val Ala Arg
               500                 505                 510
   Met Val Leu Thr Thr Glu Thr Val Val Val Asp Lys Pro Ala Lys Ala
           515                 520                 525
   Glu Asp His Asp His His His Gly His Ala His
       530                 535

## Description

The present invention relates to the use of an approximately 60kDa polypeptide (or its encoding nucleic acid molecule) or functionally equivalent molecules or fragments thereof from *Mycobacterium tuberculosis* or related prokaryotes in the prevention and/or treatment of allergic airway diseases and respiratory diseases characterised by eosinophilic inflammation and airway hyper-responsiveness.

Chaperonins are believed to stimulate the immune system at many levels simultaneously, including monocytes, macrophages, fibroblast-like cells, perhaps other types of cells, and T cells. The immune defences in mammals may be divided into the "innate" and "adaptive" defences. Those which are already in place, such as phagocytes, natural killer cells and complement are considered innate. On challenge, adaptive immunity is activated in the form of B and T lymphocytes. Chaperonins are known to act directly on the innate defence mechanisms, particularly on phagocytes. They also stimulate a powerful adaptive immune response, namely the production of antibody and the stimulation of T lymphocytes which in some cases may be protective. Notably they induce cytokine secretion which is thought to be important for host defences. In some cases however it is believed that the presence of chaperonins may be damaging to the host.

Chaperonins' role in autoimmune disease is controversial. Although infection/immunity with chaperonin-containing organisms is universal, and healthy people have T cell responses to self-chaperonins, including the production of chaperonin-specific antibodies, classical autoimmune disease is quite uncommon. So the presence of immune reactions to chaperonins may be incidental and unimportant.

The theory of molecular mimicry however suggests the involvement of chaperonins in autoimmune disease and is based on the high level of amino acid sequence conservation between chaperonins of microbial and mammalian origin. The theory proposes that during infection with a wide range of microbes, chaperonin epitopes that are shared between microbes and mammals stimulate T lymphocytes. According to this theory a high level of chaperonin presentation of shared chaperonin epitopes breaks tolerance to self-chaperonins and autoimmune disease develops.

Chaperonins obtained from tumours have been found to result in necrotic effects on those tumours. It is suggested that this may be achieved through enhancing immunological recognition of tumour antigens although the mechanism of this is not known. It therefore appears that chaperonins induce protective adaptive immunity against bacterial infection and cancer.

Allergic reactions, such as asthma, concern proportionally inappropriate or misdirected immune responses. The prevalence of asthma for example is increasing and effective therapies for treating all cases have not yet been found. Current treatment often uses immunosuppressive glucocorticosteroids, beta agonists, cromoglycate, leukotriene modifiers etc. which have numerous side-effects.

In such allergic reactions, high IgE levels occur and T helper lymphocyte-2 (Th2) immune responses predominate over Th1 responses resulting in an inflammatory response. Th1 responses are thought to be mainly protective against microbial infection and are promoted by cytokines, particularly interleukin-12 (IL-12), IL-2 and interferon-γ. In contrast, Th2 responses, in the appropriate genetic background, are associated with harmful allergic tissue damage.

However, it has been suggested that in other conditions such as autoimmune disorders, e.g. adjuvant arthritis, overactive Th1 responses are causal of the disorder. Conversion of Th1 to Th2 or Th2 to Th1 responses may therefore have utility in treating the above described disorders.

Whilst it has been known that bacteria such as *L. monocytogenes, M bovis and M tuberculosis* can convert Th2 to Th1 responses, the molecules which is(are) responsible for this conversion have not been identified.

Suggestions in the art have however implicated a heat shock protein, hsp65, from *M. leprae* which is able to induce Th1 responses (Lowrie et al., 1999, Nature, 400, p269-271; Bonato et al., 1998, Infect. Immun., 66, p169-175). The homologue, hsp65 from *M. tuberculosis,* has the ability to stimulate human monocytes to synthesize pro-inflammatory cytokines and activate monocytes and human vascular endothelial cells (Friedland et al., 1993, Clin. Exp. Immunol., 91, p5862; Peetermans et al., 1995, Infect. Immun., 63, p3454-3458; Verdegaal, et al., 1996, J. Immunol., 157, p369-376).

Surprisingly it has now been found that another protein which is not known to be a heat shock protein or a chaperonin is able to affect the immunity of an individual and can be used for treating or preventing allergic airway diseases and respiratory diseases characterised by eosinophilic inflammation and airway hyper-responsiveness.

This protein of unknown function has been identified in *Mycobacterium tuberculosis* and sequenced (Kong et al., 1993, Proc. Natl. Acad. Sci., 90, p2608-2612). Comparable proteins are known to exist in various other bacteria, including *M. bovis* and *Legionella.* It has been named chaperonin 60.1 (cpn 60.1), but adoption of this nomenclature is simply based on its amino acid sequence identity to other chaperonins.

Chaperonin 60.2 (from the same source) exhibits 59.60% amino acid sequence identity and 65.6% nucleic acid sequence identity to cpn 60.1 using the alignment methods described hereinafter. Cpn 60.2 in common with cpn 60.1 does not have confirmed chaperonin properties. Chaperonins are believed to function by the formation of 2 ring heptamers (composed of approximately 60kDa monomers) which face one another and are capped by a ring heptamer composed of approximately 10kDa monomers (formed by cpn 10s). Assisted folding is achieved once the target protein has entered into the central core, whereafter it is released. Thus the formation of the heptamers appears to be essential to the presently understood functionality of chaperonins. However, unlike cpn 60s from other species, it has not been found possible to produce heptamers of *M. tuberculosis* cpn 60.1. Furthermore, unlike the GroE chaperonin folding machinery, neither the cpn 60.1 gene nor the cpn 60.2 gene is in the same operon as the chaperonin cpn 10 gene and thus transcription of the components which are necessary for the formation of a chaperonin complex is not under the same control mechanisms.

It has also been observed that the cpn 60.1 protein has a unique histidine-rich sequence at the C-terminus unlike cpn 60s from other species which usually have a sequence rich in glycine and methionine. The 60.2 protein is a known heat shock protein and has very high homology to related heat shock proteins in other species, e.g. 95% identity to the same protein from *M*. *leprae.* As mentioned above, cpn 60.2 is situated distant to cpn 60.1 on the genome of *M. tuberculosis* and is under distinct transcriptional control. As a consequence there is no evidence to suggest that cpn 60.1 is either a heat shock protein or a chaperonin. These facts strongly suggest different functional roles for the cpn 60 proteins in *M. tuberculosis.*

The invention therefore provides molecules such as cpn 60.1 which have enhanced properties in treating or preventing allergic airway diseases and respiratory diseases characterised by eosinophilic inflammation and airway hyper-responsiveness. Therapeutic and/or prophylactic applications may be achieved using nucleic acid molecules or peptides/proteins, as will be described in more detail hereinafter.

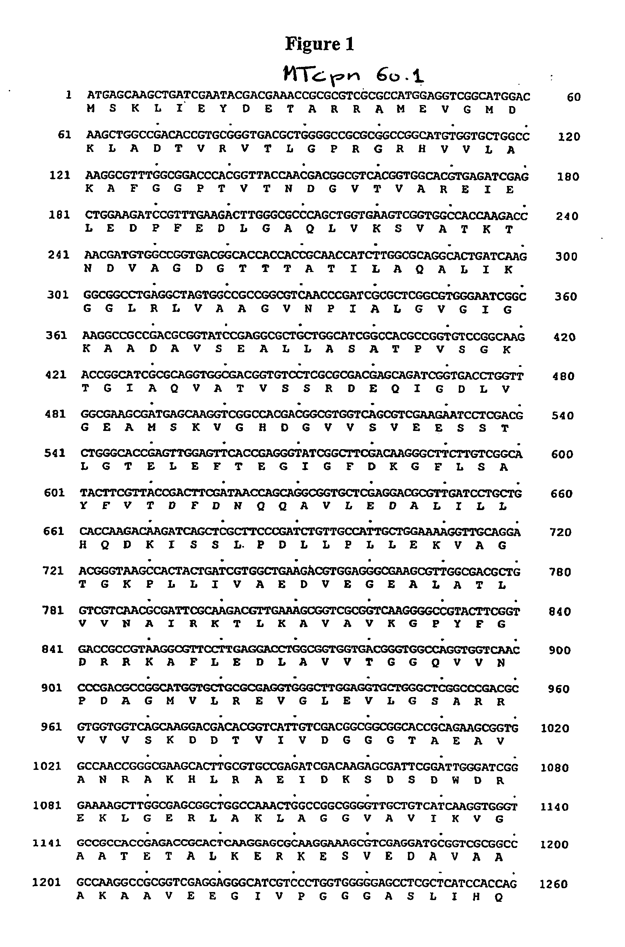

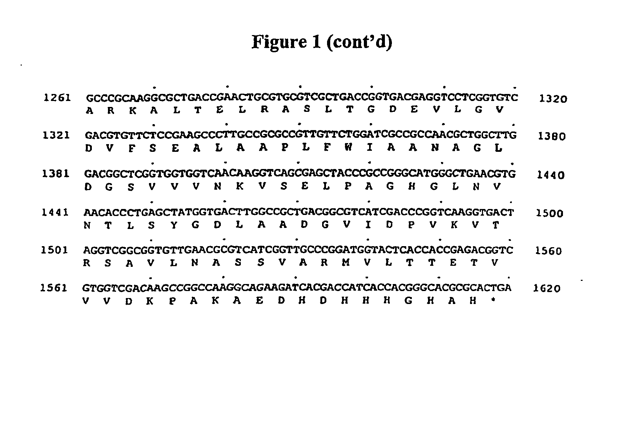

Thus, in a first aspect the present invention provides the use of pharmaceutical composition comprising a nucleic acid molecule comprising
(i) the nucleotide sequence of Figure 1, or
(ii) a sequence which has more than 66%, e.g. 70 or 75%, preferably more than 80%, e.g. more than 90 or 950% identity to sequence (i) (according to the test described hereinafter) or a sequence which hybridizes to sequence (i) under conditions of 2 x SSC, 65°C (wherein SCC = 0.15M NaCl, 0.015M sodium citrate, pH 7.2) which encodes a functionally equivalent protein to the sequence encoded by the nucleotide sequence of Figure 1, or (iii) a fragment of sequence (i) or (ii) encoding a functionally equivalent protein fragment; and a pharmaceutically acceptable excipient, diluent or carrier, for the manufacture of a medicament for the prevention and/or treatment of allergic airway diseases and respiratory diseases characterised by eosinophilic airway inflammation and airway hyper-responsiveness.

As mentioned above, therapeutic and/or prophylactic effects may be achieved using nucleic acid molecules or peptide/protein molecules. Thus in a further aspect the present invention provides the use of pharmaceutical composition comprising a polypeptide comprising
(i) the amino acid sequence of Figure 1, or
(ii) a sequence which has more than 60%, e.g. 65 or 70%, preferably more than 80%, e.g. more than 90 or 95% homology to sequence (i) (according to the test described hereinafter) which provides a functionally equivalent protein, or
(iii) a functionally equivalent fragment of sequence (i) or (ii); and a pharmaceutically acceptable excipient, diluent or carrier, for the preparation of a medicament for the prevention and/or treatment of allergic airway diseases and respiratory diseases characterised by eosinophilic airway inflammation and airway hyper-responsiveness.

"Nucleic acid molecules" according to the invention may be single or double stranded DNA, cDNA or RNA, preferably DNA. Derivatives of nucleotide sequences capable of encoding functionally-equivalent polypeptides may be obtained by using conventional methods well known in the art.

Nucleic acid molecules for use in the invention may consist only of sequences derived from Figure 1 (or related functionally equivalent sequences), or may comprise additional sequences, such as structural or functional sequences, e.g. sequences which control transcription and/or expression (particularly in mammalian cells), or sequences which comprise the sequence for an additional protein moiety which may form a fusion protein which may have specific properties e.g. act as a secretory signal. Thus, for example, the sequence may be in the form of a vector containing the nucleic acid molecules described herein. Suitable vectors include plasmids and viruses.

"Polypeptides" as referred to herein includes both full-length protein and shorter length peptide sequences, e.g. protein fragments as described herein. Such polypeptides may be prepared by any convenient means, e.g. by isolation from the source prokaryote or by recombinant means by expression of the appropriate nucleic acid molecule in a host cell operatively linked to an expression control sequence, or a recombinant DNA cloning vehicle or vector containing such a recombinant DNA molecule or by chemical or biochemical synthesis (*ex vivo*).

"Sequence identity" as referred to herein in connection with nucleotide sequences refers to the value obtained when assessed using ClustalW (Thompson et al., 1994, Nucl. Acids Res., 22, p4673-4680) with the following parameters:
Pairwise alignment parameters - Method: accurate,
Matrix: IUB, Gap open penalty: 15.00, Gap extension penalty: 6.66;
Multiple alignment parameters - Matrix: IUB, Gap open penalty: 15.00, % identity for delay: 30, Negative matrix: no, Gap extension penalty: 6.66, DNA transitions weighting: 0.5.

In connection with amino acid sequences, "sequence identity" refers to sequences which have the stated value when assessed using ClustalW (Thompson et al., 1994, supra) with the following parameters: Pairwise alignment parameters - Method: accurate,
Matrix: PAM, Gap open penalty: 10.00, Gap extension penalty: 0.10; Multiple alignment parameters - Matrix: PAM, Gap open penalty: 10.00, % identity for delay: 30, Penalize end gaps: on, Gap separation distance: 0, Negative matrix: no, Gap extension penalty: 0.20, Residue-specific gap penalties: on, Hydrophilic gap penalties: on, Hydrophilic residues: GPSNDQEKR. Sequence identity at a particular residue is intended to include identical residues which have simply been derivatized.

"Functionally equivalent" proteins or protein fragments refers to proteins or fragments related to, or derived from the amino acid sequence of Figure 1, where the amino acid sequence has been modified by single or multiple amino acid (e.g. at 1 to 50, e.g. 10 to 30, preferably 1 to 5 bases) substitution, addition and/or deletion but which nonetheless retains functional activity, e.g. suppresses ovalbumin-induced eosinophilia, for example reducing eosinophil numbers to the extent of more than 10 %, e.g. more than 25%, particularly preferably more than 50% and/or an increase in the production of specific cytokines such as interleukin-1β (IL-1β), IL-2, IL-6, IL-8, IL-10, IL-12, IL-12 receptor, tumour necrosis factor a (TNFα), interferon-γ and granulocyte-macrophage-colony stimulating factor (GM-CSF) e.g. a more than 10 fold, preferably more than 100 fold increase over normal levels and/or stimulation of Th1 responses. Cytokine stimulation can be measured by a variety of methods. For example, Buffy coat blood is diluted 3-fold with PBS-2% fetal calf serum (FCS). 30 ml is layered on 15 ml of Lymphoprep (Histopaque 1077) and centrigued at room temperature for 30 min at 700 g (1800 rpm, Eppendorf centrifuge). The layer of mononuclear cells is aspirated carefully and the cells washed twice in PBS. The cells are finally resuspended at 2 x 10⁶ cells/ml. PBMC's are subsequently seeded at 2 x 10⁶ cells/well in RPMI medium with 2% FCS, glutamine and Pen/strep and incubated for 1 h to let monocytes adhere to the plate surface. The plates are washed one with PBS.

PBMC's depleted for T cells are obtained in the same way with the sole difference of an initial incubation with the RosetteSep reagent (Stemcell) for 20 min at room temperature.

Cytokine assays are within the knowledge of skilled persons. For example, IL6 and IL8 production can be measured after diluting the cell supernatant 1/10 and 1/100 respectively. The paired antibodies and standards may be obtained from the National Institute for Biological Standards and Control and used as recommended.

Sample preparation can be as follows: Cpn60.1 and Cpn60.2 can be obtained from Lionex (Germany). Both proteins are diluted to a concentration to 200 µg/ml in PBS prior to boiling or autoclaving. The boiled samples are obtained by incubation at 100°C for 20 min and subsequently directly placed on ice. The autoclaved sample is obtained by autoclaving at 120°C for 20 min twice. SDS-PAGE can be performed on 4-20% gradient gels (Invitrogen, Netherlands). The prestained protein marker is the Benchmark Prestained Protein Ladder from Gibco/BRL. FACS analysis can be performed on a FacsCan apparatus (Becton Dickinson) and the data analysed using the WinMDI program version 2.8.

Within the meaning of "addition" variants are included amino and/or carboxyl terminal fusion proteins or polypeptides, comprising an additional protein or polypeptide fused to the polypeptide sequence.

Particularly preferred are naturally occurring equivalents such as biological variations, e.g. allelic, geographical or allotypic variants and derivatives prepared using known techniques. For example, functionally-equivalent proteins or fragments may be prepared either by chemical peptide synthesis or in recombinant form using the known techniques of site-directed mutagenesis, random mutagenesis, or enzymatic cleavage and/or ligation of nucleic acids.

The invention is particularly directed to homologues and related molecules from different prokaryotes, e.g. from bacterial genera, species or strains, particularly from the genus *Mycobacterium, e.g.* homologues from the *Mycobacterium tuberculosis* complex which includes *M. tuberculosis, M. bovis* and *M. africanum.* Such sequences may themselves be modified, particularly derivatized providing they still retain functionality.

Derivatives of the proteins may be prepared by post-synthesis/isolation modification or by modification during synthesis, e.g. using modified residues or expression of modified nucleic acid molecules, where appropriate.

Functionally-equivalent fragments according to the invention may be made by truncation, e.g. by removal of a peptide from the N and/or C-terminal ends or by selection of an appropriate active domain region, e.g. an epitopic region which retains its functionality. Such fragments may be derived from the sequence of Figure 1 or may be derived from a functionally equivalent protein to that disclosed in Figure 1.

It will be appreciated that where functional fragments are selected they may not exhibit all functions attributed to the source molecules. Thus functionally equivalent proteins or fragments refers to retention of relevant functional properties such that the fragment retains utility according to the invention, e.g. reduces eosinophilia, increases the production of specific cytokines and/or stimulates the Th1 immune response, as mentioned above.

Preferably the fragments are between 6 and 400 residues in length, e.g. 6 to 100 or 15 to 100 residues, preferably 6 to 30, 10 to 25, 15 to 50 or 15 to 30 residues. Particularly preferred fragments are those derived from or consisting of residues:

| | |
|---|---|
| 1-8 | MSKLIEYD, (8) |
| 14-21 | AMEVGMDK, (8) |
| 40-48 | AKAFGGPTV, (9) |
| 64-71 | PFEDLGAQ, (8) |
| 96-105 | QALIKGGLRL, (11) |
| 110-129 | VNPIALGVGIGKAADAVSEA, (20) |
| 132-143 | ASATPVSGKTGI, (12) |
| 144-155 | AQVATVSSRDEQ, (12) |
| 160-175 | VGEAMSKVGHDGVVSV, (16) |
| 179-200 | STLGTELEFTEGIGFDKGFLSA, (22) |
| 195-219 | KGFLSAYFVTDFDNQQAVLEDALIL, (25) |
| 206-219 | FDNQQAVLEDALIL, (14) |
| 221-229 | HQDKISSLP, (9) |
| 264-271 | AIRKTLKA, (8) |
| 276-293 | GPYFGDRRKAFLEDLAVV, (18) |
| 299-314 | VNPDAGMVLREVGLEV, (16) |
| 315-326 | LGSARRVVVSKD (12) |
| 327-342 | DTVIVDGGGTAEAVAN, (16) |
| 343-353 | RAKHLRAEIDK, (11) |
| 379-391 | VGAATETALKERK (13) |
| 392-400 | ESVEDAVAA, (9) |
| 411-433 | PGGGASLIHQARKALTELRASLT, (23) |
| 434-449 | GPEVLGVDVFSEALAA, (16) |
| 450-463 | PLFWIAANAGLDGS, (14) |
| 464-471 | VVVNKVSE, (8) |
| 480-494 | VNTLSYGDLAADGVI, (15) |
| 501-526 | RSAVLNASSVARMWTTETVVVDKPA, (15) |
| 526-539 | KAEDHDHHHGHAH. (14) |

Functionally equivalent nucleic acid sequences/fragments compared to the sequence recited in Figure 1 are also used in compositions of the invention. These sequences are defined with reference to the functionally equivalent protein/peptides (as defined above) which they encode.

"Hybridisation" as used herein refers to those sequences which bind under non-stringent conditions (6 x SSC/50% formamide at room temperature) and washed under conditions of high stringency e.g. 2 x *SSC,* 65°C (where *SSC* = 0.15M NaCl, 0.015M sodium citrate, pH 7.2).

"Pharmaceutically acceptable" as referred to herein refers to ingredients that are compatible with other ingredients of the compositions as well as physiologically acceptable to the recipient.

Pharmaceutical compositions according to the invention may be formulated in conventional manner using readily available ingredients. Thus, the active ingredient (ie. the nucleic acid molecule or protein/peptide), may be incorporated, optionally together with other active substances, with one or more conventional carriers, diluents and/or excipients, to produce conventional galenic preparations such as tablets, pills, powders, lozenges, sachets, cachets, elixirs, suspensions, emulsions, solutions, syrups, aerosols (as a solid or in a liquid medium), ointments, soft and hard gelatin capsules, suppositories, sterile injectable solutions, sterile packaged powders, and the like.

As mentioned above, compositions may additionally comprise molecules which assist or augment the action of the nucleic acid molecules or polypeptides described hereinbefore, e.g. thalidomide (and analogues thereof), low dose cyclophosphamide, LPS, cytokines, chemokines, CpG oligodeoxynucleotides and other immunomodulators and/or antiinflammatory agents such as cytokine antagonists or glucocorticosteroids.

Thus for example, the compositions may be used together with active ingredients for specific immunotherapies. Appropriate immunotherapy treatment/vaccine preparations which may include nucleic acid molecules/polypeptides as described herein include subunit vaccines or treatments based on cell specific antigens or associated antigens or antibody, anti-idiotype antibody or whole cell preparations for vaccination or therapy. When used in therapy or vaccination the nucleic acid molecules or polypeptides described herein may provide (or encode) an antigen resulting in a specific immune response directed to that antigen and/or may result in a general and nonspecific immune response. In the latter case in which compositions containing other active ingredients are used, the nucleic acid molecules/polypeptides described herein act as adjuvants and may be used for this purpose.

Preventative or therapeutic preparations may be formulated to include one or more suitable adjuvants, e.g. Incomplete Freund's Adjuvant, BCG, Montanide, aluminium hydroxide, saponin, quil A, or more purified forms thereof, muramyl dipeptide, mineral or vegetable oils, Novasome or non-ionic block co-polymers or DEAE dextran, in the presence of one or more pharmaceutically acceptable carriers or diluents. Suitable carriers include liquid media such as saline solution.

Examples of suitable carriers, excipients, and diluents are lactose, dextrose, sucrose, sorbitol, mannitol, starches, gum acacia, calcium phosphate, aglinates, tragacanth, gelatin, calcium silicate, microcrystalline cellulose, polyvinylpyrrolidone, cellulose, water syrup, water, water/ethanol, water/glycol, water/polyethylene glycol, propylene glycol, methyl cellulose, methylhydroxybenzoates, propyl hydroxybenzoates, talc, magnesium stearate, mineral oil or fatty substances such as hard fat or suitable mixtures thereof. The compositions may additionally include lubricating agents, wetting agents, emulsifying agents, suspending agents, preserving agents, sweetening agents, flavouring agents, and the like. The compositions of the invention may be formulated so as to provide quick, sustained or delayed release of the active ingredient after administration to the patient by employing procedures well known in the art.

Compositions may be in an appropriate dosage form, for example as an emulsion or in liposomes, niosomes, microspheres, nanoparticles or the like.

If required, the compositions may also contain targeting moieties attached to the active ingredient, e.g. a ligand which binds specifically and selectively to an endogenous receptor to allow targeting to a particular cell type or location, such as targeting to lymphocytes, monocytes, macrophages, endothelial cells, epithelial cells, blood cells, erythrocytes, platelets, eosinophils, neutrophils, natural killer cells, dendritic cells, brain cells, heart cells, lung cells, islet cells, kidney cells, hormonal gland cells, skin, bone, joints, bone marrow, gastric mucosa, lymph nodes, Peyers patches, the omentum and other immunological tissues.

The above described compositions have utility in the treatment or prophylaxis of non-cancerous conditions such as autoimmune disorders or conditions such as conditions of immunoactivation, allergic reactions and/or conditions typified by a Th2-type immune response and/or conditions associated with eosinophilia.

Alternatively viewed, the present invention provides a use for treating or preventing allergic airway disease and respiratory diseases characterised by eosinophilic airway inflammation and airway hyper-responsiveness wherein said patient is to be administered a pharmaceutical composition as described hereinbefore. Furthermore, the present invention provides the use of a nucleic acid molecule comprising
(i) the nucleotide sequence of Figure 1, or
(ii) a sequence which has more than 66%, e.g. 70 or 75%, preferably more than 80%, e.g. more than 90 or 95% identity to sequence (i) (according to the test described hereinbefore) or a sequence which hybridizes to sequence (i) under conditions of 2 x SSC, 65'C (wherein SCC = 0.15M NaCl, 0.015M sodium citrate, pH 7.2) which encodes a functionally equivalent protein to the sequence encoded by the nucleotide sequence of Figure 1, or (iii) a fragment of sequence (i) or (ii) encoding a functionally equivalent protein fragment; or
   a polypeptide comprising
   (i) the amino acid sequence of Figure 1, or
   (ii) a sequence which has more than 60%, e.g. 65 or 70%, preferably more than 80%, e.g. more than 90 or 95% homology to sequence (i) (according to the test described hereinbefore) which provides a functionally equivalent protein, or
   (iii) a functionally equivalent fragment of sequence (i) or (ii);
   in the preparation of a medicament for treating or preventing a non-cancerous condition, such as autoimmune disorders or conditions of immunoactivation, allergic responses and/or conditions typified by a Th2-type immune response and/or conditions associated with eosinophilia.

As defined herein "treatment" refers to reducing, alleviating or eliminating one or more symptoms of the condition which is being treated, relative to the symptoms prior to treatment. For example, symptoms which may be affected include eosinophilia, decreased secretion of particular cytokines, a Th2-biased immune response, allergic response, presence of autoantibodies, etc which are treated to achieve the effects particularly as defined in respect of the functional properties of functionally equivalent polypeptides.

"Prevention" of a condition refers to delaying or preventing the onset of a condition or reducing its severity, as assessed by the appearance or extent of one or more symptoms of said condition.

Allergic conditions which may be treated or prevented include allergic rhinitis, allergic airway diseases, hyper-eosinophilic syndrome, contact dermatitis, and respiratory diseases characterized by eosinophilic airway inflammation and airway hyperresponsiveness, such as allergic asthma, intrinsic asthma, allergic bronchopulmonary aspergillosis, eosinophilic pneumonia, allergic bronchitis bronchiectasis, occupational asthma, reactive airway disease syndrome, interstitial lung disease and hypereosinophilic syndrome. Preferably however the composition is used for treating asthma. In a further preferred feature, the composition is used for treating respiratory conditions in which eosinophilia plays a role, e.g. allergies (as described above, particularly asthma), and pulmonary eosinophilia.

Patients which may be treated include, but are not limited to mammals, particularly primates, domestic animals and livestock. Thus preferred animals for treatment include mice, rats, guinea pigs, cats, dogs, pigs, goats, sheep, horses and particularly preferably, humans.

As mentioned previously, either nucleic acid molecules or polypeptides may be used in the methods of the invention. In instances in which nucleic acid molecules are employed, these are conveniently applied in a form to allow their expression within the patient, thus providing a form of gene therapy. Thus the pharmaceutical compositions described herein containing a nucleic acid molecule may be used in methods of gene therapy.

Thus for example the nucleic acid molecules may be provided in a liposome, micelle or other convenient carrying vehicle which may comprise targeting moieties to allow its targeting to cells of interest.

Alternatively the molecules may be packaged in other, "vehicles" such as viruses, plasmids or cells (particularly transfected species-matched cells) which are all well known in the art for this purpose which allow expression of the resident molecule.

Appropriate techniques for transfection are well known and include electroporation, microinjection, lipofection, adsorption, viral transfection and protoplast fusion.

Administration of compositions of the invention may take place by any of the conventional routes, e.g. by inhalation, nasally, orally, rectally or parenterally, such as by intramuscular, subcutaneous, intraperitoneal or intravenous injection. Treatment or prophylaxis by topical application of a composition, e.g. an ointment, to the skin is also possible. Optionally administration may be performed at intervals, e.g. 2 or more applications, e.g. 2-4 applications at hourly, daily, weekly or monthly intervals, e.g. several times a day, or every 3-5 days, or at fortnightly, monthly or quarterly intervals.

It has been observed in work conducted on the related molecule cpn 60.2 that the route of administration may affect the immune response which is generated. For example when Mtcpn 60.2 is administered intranasally, a Th2 to Th1 shift is stimulated although the reverse effect is observed when administered intraperitoneally. Thus, the route of administration should take into account the disorder to be treated/prevented and thus for example in treating autoimmune disorders, intraperitoneal administration may be appropriate whereas treatment or prevention of particularly allergic disorders may be for example by intranasal administration.

In prophylactic methods of the invention, administration (conveniently orally or by inhalation or subcutaneous or intramuscular injection) is preferably performed at more lengthy intervals, e.g. intervals of 2-12 weeks. For therapeutic purposes, administration (conveniently orally or by inhalation or intravenous injection) is performed 1-4 times in a single day or over 2 days.

The active ingredient in composition of the invention may comprise from about 0.01% to about 99% by weight of the formulation, preferably from about 0.1 to about 50%, for example 10%. The compositions are preferably formulated in a unit dosage form, e.g. with each dosage containing from about 0.01mg to about 1g of the active ingredient, e.g. 0.05mg to 0.5g, for a human, e.g. 1-100mg.

The precise dosage of the active compound to be administered and the length of the course of treatment will, of course, depend on a number of factors including for example, the age and weight of the patient, the specific condition requiring treatment and its severity, and the route of administration. Generally however, an effective dose may lie in the range of from about 0.1µg/kg to about 14mg/kg, preferably 0.1 to 1mg/kg, e.g. from about 1mg to 1g of polypeptide per day, depending on the animal to be treated and the dosage form, taken as a single dose. Thus for example, an appropriate daily dose for an adult may be from 7µg to 1g, e.g. 10mg to 1g per day, e.g. 25 to 500mg of the polypeptide per day.

Similar or lower dosages may be used when using nucleic acid molecules described herein, e.g. from about 0.2ng/kg to about 2.5mg/kg (e.g. from about 0.2ng/kg to about 2ng/kg or about 1.5ng/kg to about 2.5mg/kg) such as about 14ng to about 175mg for an adult. However, where the nucleic acid molecules are packaged in cells or vectors proportionally higher or lower amounts may be required depending on the extent of non-cpn encoding DNA and sequences which influence the level of expression, e.g. 5 or 10-fold larger amounts, e.g. nucleic acid molecules described herein packaged in a vector may be used at about 1.0ng/kg to about 12.5mg/kg.

As mentioned above, the family of polypeptides defined herein and the nucleic acid molecules encoding them stimulate the production of a set of cytokines. This therefore allow the use of these compounds for the express purpose of stimulating production of these cytokines whether or not this occurs in a therapeutic/prophylactic situation. Thus in a further aspect the present invention provides a method of stimulating cytokine production in a cell, wherein said method comprises administration of
a nucleic acid molecule comprising
(i) the nucleotide sequence of Figure 1, or
(ii) a sequence which has more than 66%, e.g. 70 or 75%, preferably more than 80%, e.g. more than 90 or 95% identity to sequence (i) (according to the test described hereinbefore) or a sequence which hybridizes to sequence (i) under conditions of 2 x SSC, 65°C (wherein SCC = 0.15M NaCl, 0.015M sodium citrate, pH 7.2) which encodes a functionally equivalent protein to the sequence encoded by the nucleotide sequence of Figure 1, or (iii) a fragment of sequence (i) or (ii) encoding a functionally equivalent protein fragment; or a polypeptide comprising

(i) the amino acid sequence of Figure 1, or
(ii) a sequence which has more than 60%, e.g. 65 or 70%, preferably more than 80%, e.g. more than 90 or 95% homology to sequence (i) (according to the test described hereinbefore) which provides a functionally equivalent protein, or
(iii) a functionally equivalent fragment of sequence (i) or (ii); to said cell.

Such methods may be performed *in vitro,* e.g. on cells, tissues or organs outside the body. This methodology may for example be used in research methods to identify the molecule or molecules which react or bind to or are activated via molecules of the invention, e.g. cpn 60.1 receptor molecules As a corollary to such methods, the stimulation of cytokine production may be used to measure the presence of molecules of the invention.

Thus, in a further aspect the present invention provides a method of assessing the presence or concentration of a polypeptide or peptide of the invention in a sample wherein said sample is applied to a cell and the level of production of one or more cytokines is measured and compared to the level of production of said one or more cytokines in a control sample wherein the increase over control levels provides a correlation to the presence or concentration of said polypeptide or peptide in said sample.

As used herein "control" refers to a sample which does not contain molecules of the invention or moieties which increase production of the cytokine(s) to be measured. Where appropriate, standard curves may be generated using molecules of the invention to allow quantitative assessment to be made of the presence or concentration of said molecules, although qualitative assessments may also be made. This method may furthermore be used to identify molecules of the invention.

Alternatively however, the method of stimulating cytokine production may be performed *in vivo* to enhance production of particular cytokines. This may have beneficial therapeutic or prophylactic effects and in which case the invention extends to the nucleic acid molecules and polypeptides as described above for use in treating conditions which may be alleviated, overcome or prevented by increasing specific cytokines, and the use of such molecules for the preparation of medicaments for that purpose.

Preferably the cytokines which are increased, e.g. more than 10 or 100 fold relative to normal levels, are selected from the group consisting of IL-1β, IL-2, IL-6, IL-8, IL-10, IL-12, TNFα, interferon-γ and GM-CSF.

### Definitions

"AUTOIMMUNE DISEASE". This term intended to cover those cases where it can be shown that the autoimmune process contributes to the pathogenesis of a disease. Such diseases are typically associated with a T helper lymphocyte-1 (Th-1) type immune response.
"ALLERGIC CONDITIONS". This term is intended to cover conditions associated with a T helper lymphocyte-2 (Th-2) type immune response. In allergic reaction, high IgE levels occur and Th-2 immune responses predominate over Th-1 responses, resulting in inflammatory response. Examples of allergic conditions include the following: asthma, rhinitis/hay fever, eczema and anaphylaxis.
"ADJUVANT". This term is intended to cover any substance which, when incorporated into or administered simultaneously with antigen, potentiates the immune response.
"MT60.1", "Mtcpn60.1", "cpn 60.1", "60.1" are used interchangeably throughout the specification to refer to the amino acid sequence shown in Figure 1.

### Description of Figures

Figure 1 shows the nucleotide and amino acid sequence of cpn 60.1 from *M. tuberculosis;*
Figure 2 shows the *in vitro* effects of *M. tuberculosis* cpn 60.1 and 60.2 on cytokine production; A) IL-1β, B) IL-6, C) IL-8, D) IL-10, E) IL-12, F) TNFα and G) GM-CSF, wherein the filled circles represent cpn 60.1 and the open circles represent cpn 60.2;
Figure 3 show the effects of BCG on airway inflammation in mice; and
Figure 4 shows the reduction in eosinophil levels in mice with ovalbumin-induced pulmonary eosinophilia after the administration of 5 doses of cpn 60.1;
Figure 5 shows that the percentage of eosinophils found in bronchoalveolar lavage fluid in ovalbumin immunized mice (ova) was 37.7±7.4%.
Figure 6 shows ELISA results which indicate a significant antibody generation in chaperonin-treated rats (ie 60.1 and 60.2 treated animals), but not in untreated, or PBS treated animals. Key: Naive (no immunisation); Antigen used in ELISA is cpn 60.2 ("65"); cpn 60.1 ("cpn") or cpn 10 ("10"). For example, on the y axis "Naïve/65" indicates that the animal was not immunised and the antigen used in the ELISA was 60.2. Similarly, "cpn/cpn" indicates that the animal was immunised with 60.1 and that the antigen used in the ELISA was 60.1.

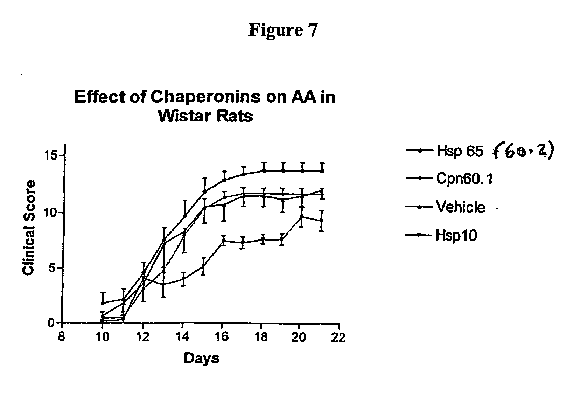

Figure 7 shows the effect, in terms of clinical score for arthritis, of treatment with and without cpn60.1, 60.2 or 10.
Figure 8 shows the effect, in terms of x-ray score for arthritis, of treatment with and without cpn60.1, 60.2, or 10. Key: "Ost" = osteoporosis; "Peri" = perioditis; and "Ero" = erosion.

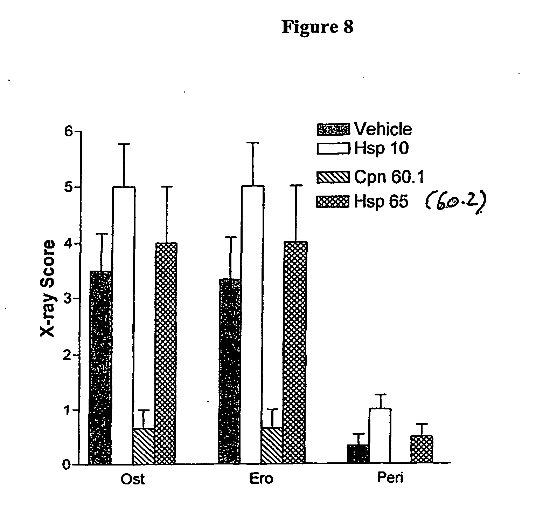

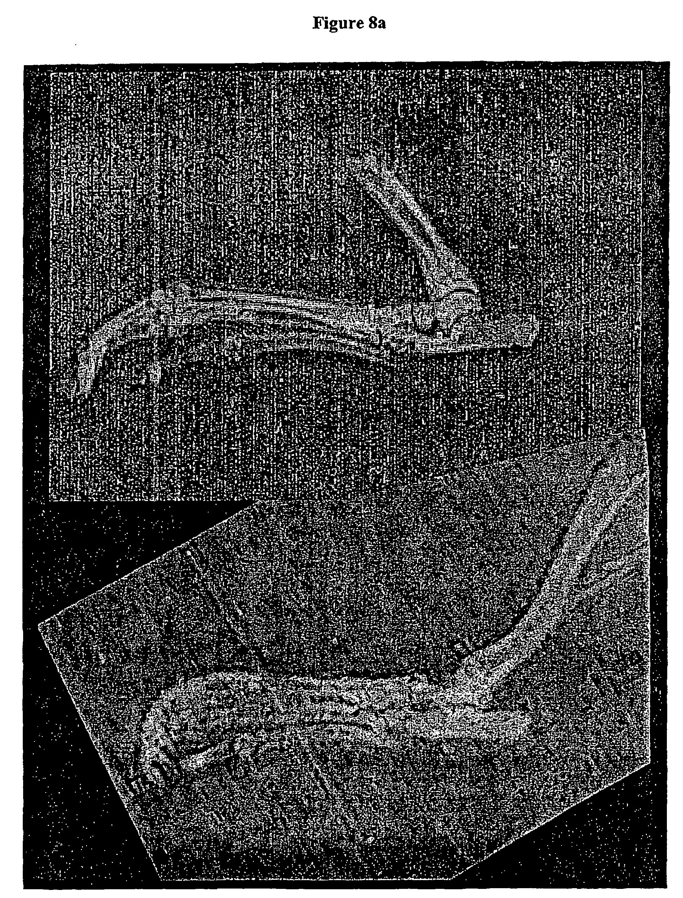

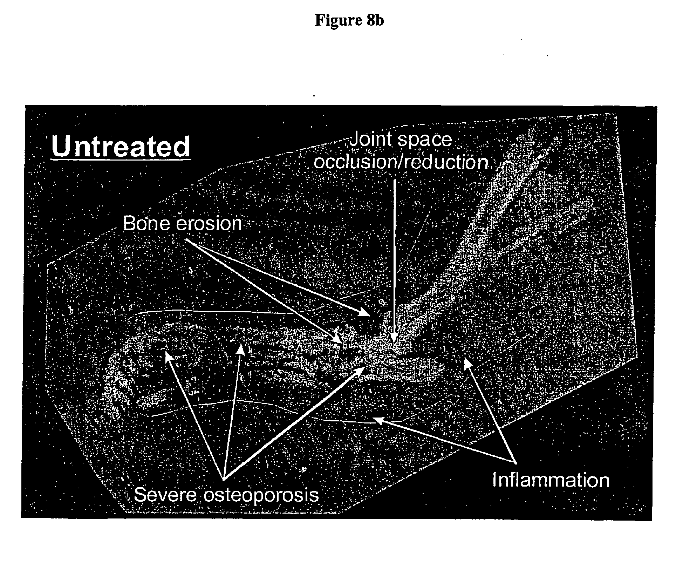

Figure 8(a) shows the effect of cpn60.1 treatment of adjuvant-induced rheumatoid arthritis in the Wistar rat. Arthritis was induced by a single intra-dermal tail injection of heat-killed *Mycobacterium tuberculosis* in oil (adjuvant). Treatment was by injection of CPN 60.1 (50 µg in phosphate buffered saline) on days 4, 5 and 6 after induction. Disease progression, assessed on paw inflammation, was monitored daily. X-ray scans were taken when arthritis was maximal (>9 days following treatment). Upper scan: rat rear paw treated with CPN 60.1 post induction with adjuvant, showing bone density and joint physiology indistinguishable from normal rats. Lower scan: rat rear paw showing rheumatoid lesions typical in adjuvant-induced arthritis: bone erosion, osteoporosis, joint changes and occlusion. These are annotated in Figure 8b.
Figures 9 and 10 show the relative levels of antibody production in rats immunised with 60.1 (Figure 9), 60.2 (Figure 10) and in PBS treated animals.

### EXAMPLE 1: Mycobacterium tuberculosis cpn 60.1 is a powerful inducer of cytokines

In this experiment the cytokine inducing activity of purified *M. tuberculosis* cpn 60.1 recombinant proteins were examined by ELISA.

### Methods

*Expression and purification of chaperonin 60 proteins M. tuberculosis* cpn 10, 60.1 and 60.2 were prepared by Prof M. Singh (WHO Collaborating Centre, Germany) using conventional chromatography as described below.

### Purification of recombinant cpn 60.2:

The protein was obtained from heat-induced (42°C) recombinant *E.coli* K12 cells that carry a plasmid encoding *M. tuberculosis* cpn 60.2. Cells were lysed by sonication. The supernatant was chromatographed on an anion exchange chromatography column. After dialysis the fractions containing cpn 60.2 were further purified on a second different anion exchange chromatography column. Finally the protein solution was dialysed against 10mM ammonium bicarbonate before aliquotting and lyophilization..

Great care was taken to check each batch of protein for LPS contamination using the Limulus assay (Tabona et al., 1998, J. Immunol., 161, p1414-1421). If LPS contamination was detected it was removed on a polymyxin B affinity column and levels of LPS re-assayed. Recombinant, LPS-low, chaperonins were further purified on a Reactive Red column to remove contaminating proteins and peptides (Tabona et al., 1998, supra).

The *in vitro* effects of cpn 60.1 and cpn 60.2 on the production of IL-1β, IL-6, IL-8, IL-10, IL-12, TNFα and GM-CSF in human PBMCs was determined using 2-site ELISA as described by Tabona et al., 1998, supra.

### Results

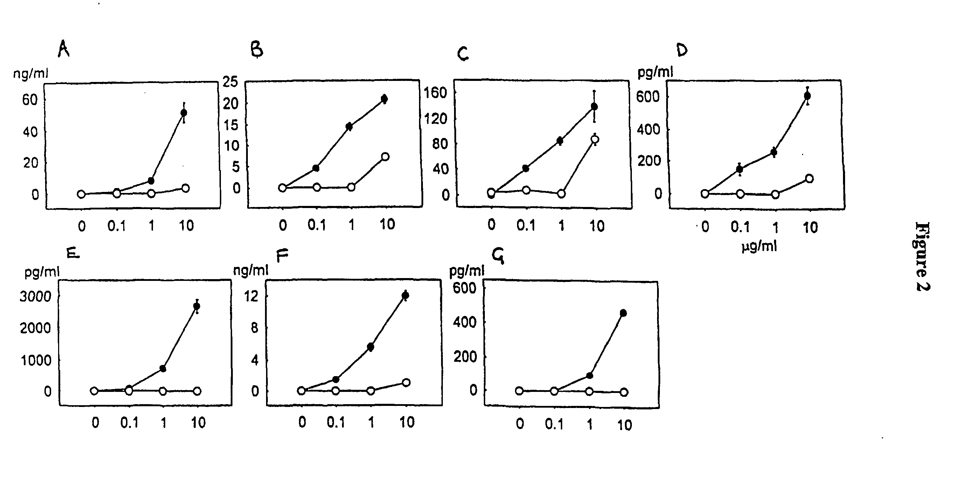

The results are shown in Figure 2. Surprisingly, the cpn 60.1 protein proved to be a much more potent cytokine inducer than the well-studied cpn 60.2 or hsp65. In addition to being two log orders more potent than cpn 60.2, the cpn 60.1 protein stimulates a significantly greater maximal response in human monocytes than does cpn 60.2 or LPS. Of interest, both chaperonins stimulate IL-12 production but fail to promote the formation of the anti-mycobacterial cytokine IFN-γ. In this context we have examined a number of cpn 60-derived peptides. The putative cpn 60.1 T cell epitope peptide, 195-219, proved to be a potent inducer of cytokine synthesis, including IFN-γ (data not shown). The same peptides in *M. tuberculosis* cpn 60.2 and in the *E. coli* cpn 60 protein, groEL, were without cytokine-inducing activity (Lewthwaite, Henderson and Coates, unpublished data). These findings confirm the action of *M. tuberculosis* cpn 60.1 on monocytes/macrophages and thus their use in the prevention or treatment of certain conditions.

### EXAMPLE 2: Mycobacterium tuberculosis cpn 60.1 suppresses asthma in the mouse

This Example shows for the first time that in a murine model of allergic inflammation *M. tuberculosis* cpn 60.1 protein inhibited the recruitment of eosinophils to the airways in immunized mice. The effect of cpn 60.1 on the eosinophilic response is dependent on the dose and timing. These data show that Mtcpn 60.1 modulates airway inflammation in the mouse and therefore, has important implications for allergic disease treatment and prevention.

### Methods

*Murine Model of Inflammation -* A murine model of allergic inflammation that allows the quantitation of eosinophil and T lymphocyte recruitment in the airways following antigen challenge has been developed. Furthermore, a pulmonary monitoring system is used which allows changes in pulmonary mechanics to bronchoconstrictor agonists *in vivo* to be determined.

*Immunisation Protocol -* C57B1/6 wild type (local supplier) 6 - 8 weeks old mice were immunised with ovalbumin (10 µg intraperitoneal injection; in 1 mg aluminium hydroxide) on day 0 and repeated 7 days later. On days 14, 15 and 16 mice were placed in a plexiglass container (12 L) and exposed to a nebulised solution of ovalbumin (10 mg/ml; De Vilibiss Ultraneb 90). Sham immunised wild type mice were injected with (1 mg aluminium hydroxide) on days 0 and 7 and also challenged with ovalbumin on days 14-16. Aerosol exposure was performed by exposure 3 times daily for 20 min at hourly intervals and bronchoalveolar lavage, collection of lungs for immunohistochemistry and lung mechanics was performed 24 h after the last aerosol challenge.

While ovalbumin is not a respiratory allergen often encountered by asthmatic subjects, the Th2 responses observed in murine models of inflammation are analogous to those observed following immunization with house dust mite. The Th2 cytokine profile generated by both allergens are similar. The advantage of using ovalbumin is that it is easily available and the specific activity of this allergen does not change between batches and therefore, we can control for antigen dose between batches.

*BCG treatment:* Six days following immunization with ovalbumin (10µg), mice were injected with BCG (log BCG viable units: -4, -5 and -6) via the intravenous route. On day 7, mice received a booster injection with ovalbumin (10µg). On day 13, mice received a second administration of BCG at the same dose and route as described for day 6. On day 14, mice were placed in a plexiglass box and exposed three times with nebulized ovalbumin (1% solution) for a period of 30 minutes at 1 hour intervals. This procedure was repeated on day 15 and 16. 24 hours after the last ovalbumin exposure, mice were anaesthetised and a bronchoalveolar lavage performed for the enumeration of eosinophils.

*Cpn 60.1*/*60.2 treatment:* Mice from the same batch of animals were immunized to ovalbumin and treated with Mtcpn 60.1 (10µg/animal) by direct instillation into the trachea. Mice were treated with Mtcpn 60.1 on day 6 and day 13 and then 30 min before the commencement of the challenge protocol on days 14, 15, and 16 (a total of 5 treatments).

### Results

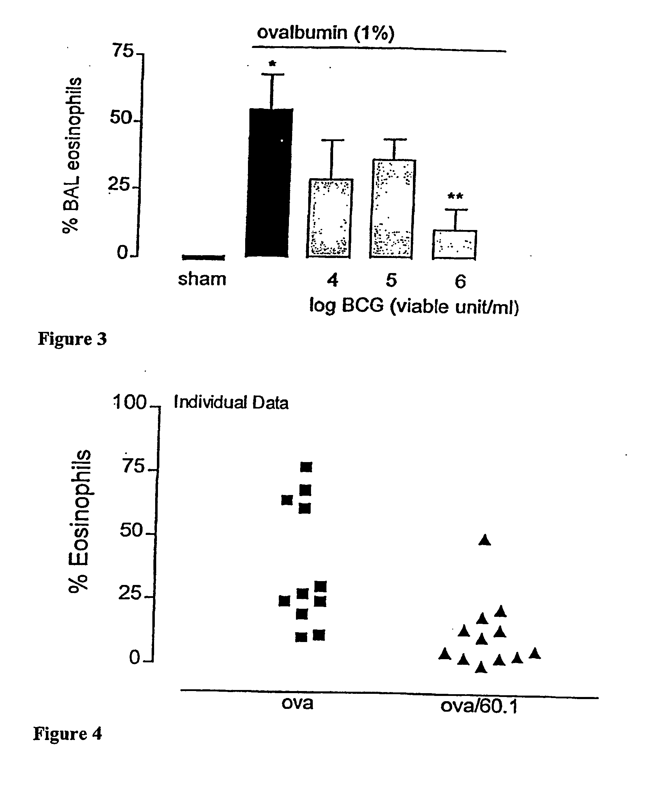

Figure 3 shows a significant recruitment of eosinophils to the airways in ovalbumin- but not sham-immunized mice.

Figure 3 also shows that BCG suppresses airways inflammation in mice. In ovalbumin immunized mice, ovalbumin challenge induces a pulmonary eosinophilia (55 ± 12 %, n = 5, P < 0.05 cf sham), which is significantly suppressed in mice treated with BCG (10⁶ viable units/ml) prior to antigen.

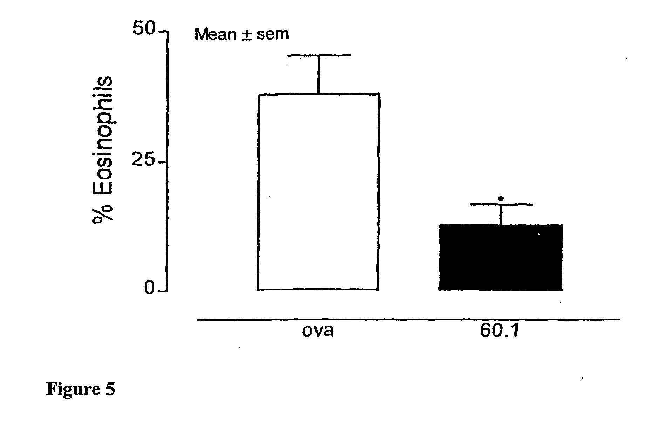

Figure 4 shows the results obtained with and without treatment with cpn 60.1. The percentage of eosinophils found in bronchoalveolar lavage fluid in ovalbumin immunized mice (ova) was 37.7 ± 7.4 % (Figure 5). There was significant suppression of the recruitment of eosinophils to the airways following ovalbumin challenge (% eosinophils in Mtcpn 60.1 treated mice; 12.6 ± 3.9 % P< 0.05 cf control). This provides strong evidence of a protective effect of *M. tuberculosis* in asthma.

The applicants have also performed another experiment whereby mice were treated with Mtcpn 60.1 on only two occasions (day 6 and day 13) and found that eosinophil recruitment was not inhibited. This indicates that the effect of Mtcpn 60.1 on the eosinophilic response is dependent on the dose and timing.

These data demonstrate for the first time that Mtcpn 60.1 can suppress eosinophilic inflammation in a murine model of asthma. Other allergic conditions such as rhinitis/hay fever, eczema and anaphylaxis share a common mechanism (over-reactivity of Th-2 cells) with asthma. Hence, if 60-1 can inhibit asthma, it should inhibit other allergic conditions. This supports the hypothesis that this protein has the potential to modulate airways inflammation in the mouse, which has important implications for the treatment and prevention of non-cancerous conditions such as allergic diseases and autoimmune diseases.

An important advantage of 60.1 is that it can provide a prophylactic therapy as distinct from a treatment of acute symptoms of an allergic condition. In other words, 60.1 treatment can prevent the acute symptoms of an allergic condition from developing.

Example 4: Adjuvant Activity - Antibody production in normal and adjuvant activity (AA) rats given a subcutaneous injection at the base of the tail of a preparation containing cpn 60.1 and 60.2 in PBS (buffer) only and in naive rates (no immunisation). The experimental details are the same as those described in Example 3.

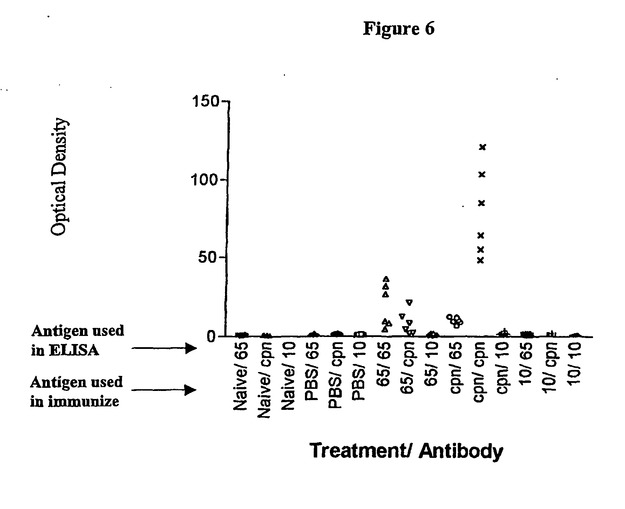

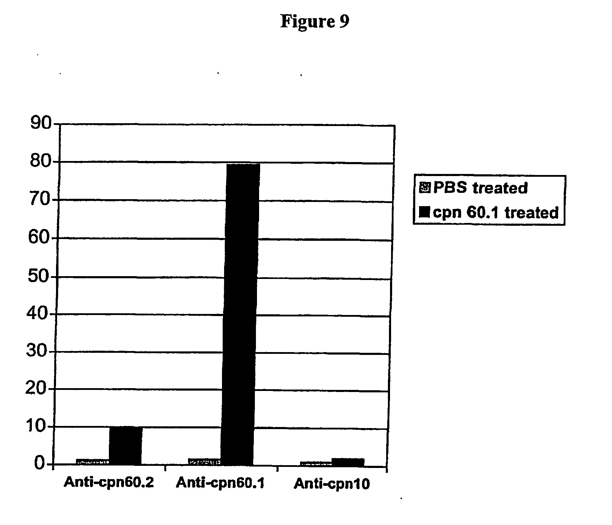

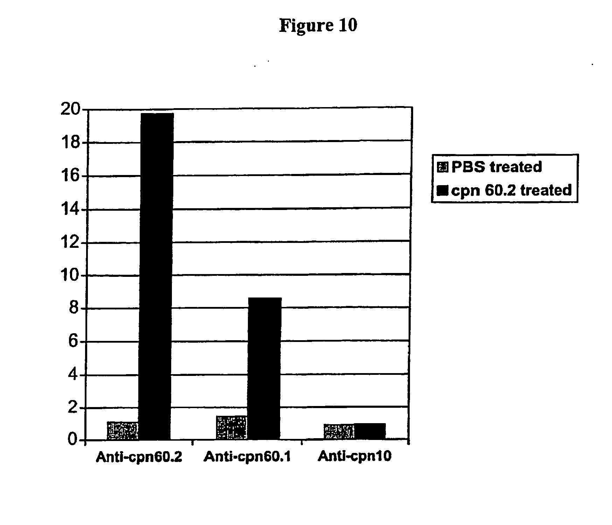

Figures 6, 9 and 10 show a significant production of antibodies in rats treated with 60.1 and 60.2, but not in PBS-treated animals or naïve animals.

Hence, 60.1 and 60.2 possess a very strong adjuvant activity. Still further, 60.1 produced four times the antibody response produced by 60.2 Accordingly, 60.1 can act as an adjuvant for antigens in vaccine compositions. Particularly preferred examples of vaccine compositions comprise the compound of the invention together with one or more of the following antigens:

Anthrax, Cholera, Diphtheria, Haemophilus influenza b (Hib), Hepatitis A, Hepatitis B, Influenza, Japanese encephalitis, Measles, mumps and rubella (MMR), Meningococcal, Pertussis, Pneumococcal, Poliomyelitis (sabin and salk vaccines), Rabies, Rubella, Smallpox and vaccinia, Tetanus, Tick borne encephalitis, Tuberculosis (BCG), Typhoid, Varicella/herpes zoster, Yellow fever and antigens for veterinary vaccines, eg foot and mouth disease virus.

## Claims

1. The use of a pharmaceutical composition in the manufacture of a medicament for the prevention and/or treatment of allergic airway diseases and respiratory disease **characterised by** eosinophilic airway inflammation and airway hyper-responsiveness wherein the pharmaceutical composition comprises a nucleic acid molecule comprising
(i) the nucleotide sequence of Figure 1, or
(ii) a sequence which has more than 66%, e.g. 70 or 75% preferably more than 80%, e.g. more than 90 or 95% identity to sequence (i) or a sequence which hybridizes to sequence (i) under conditions of 2 x SSC, 65°C (wherein SCC = 0.15M NaCl, 0.015M sodium citrate, pH 7.2) which encodes a functionally equivalent protein to the sequence encoded by the nucleotide sequence of Figure 1, or
(iii) a fragment of sequence (i) or (ii) encoding a functionally equivalent protein fragment; and a pharmaceutically acceptable excipient, diluent or carrier.

2. The use of a pharmaceutical composition in the manufacture of a medicament for the prevention and/or treatment of allergic airway diseases and respiratory disease **characterised by** eosinophilic airway inflammation and airway hyper-responsiveness wherein the pharmaceutical composition comprises a polypeptide comprising
(i) the amino acid sequence of Figure 1, or
(ii) a sequence which has more than 60%, e.g. 65 or 70%, preferably more than 80%, e.g. more than 90 or 95% homology to sequence (i) which provides a functionally equivalent protein, or
(iii) a functionally equivalent fragment of sequence (i) or (ii); and
a pharmaceutically acceptable excipient, diluent or carrier.

3. The use as claimed in Claim 2 wherein the fragments are between 6 and 400 residues in length.

4. The use as claimed in Claim 3 wherein the fragment lengths are between 6 to 100 or 15 to 100 residues.

5. The use as claimed in Claim 4, wherein the fragment lengths are between 6 to 30, 10 to 25, 15 to 50 or 15 to 30 residues.

6. The use as claimed in Claim 2 wherein the fragments are derived from or consisting of at least one of the following residues:
| | |
|---|---|
| 1-8 | MSKLIEYD, (8) |
| 14-21 | AMEVGMDK, (8) |
| 40-48 | AKAFGGPTV, (9) |
| 64-71 | PFEDLGAQ, (8) |
| 96-105 | QALIKGGLRL, (11) |
| 110-129 | VNPIALGVGIGKAADAVSEA, (20) |
| 132-143 | ASATPVSGKTGI, (12) |
| 144-155 | AQVATVSSRDEQ, (12) |
| 160-175 | VGEAMSKVGHDGVVSV, (16) |
| 179-200 | STLGTELEFTEGIGFDKGFLSA, (22) |
| 195-219 | KGFLSAYFVTDFDNQQAVLEDALIL, (25) |
| 206-219 | FDNQQAVLEDALIL, (14) |
| 221-229 | HQDKISSLP, (9) |
| 264-271 | AIRKTLKA, (8) |
| 276-293 | GPYFGDRRKAFLEDLAVV, (18) |
| 299-314 | VNPDAGMVLREVGLEV, (16) |
| 315-326 | LGSARRVVVSKD, (12) |
| 327-342 | DTVIVDGGGTAEAVAN, (16) |
| 343-353 | RAKHLRAEIDK, (11) |
| 379-391 | VGAATETALKERK, (13) |
| 392-400 | ESVEDAVAA, (9) |
| 411-433 | PGGGASLIHQARKALTELRASLT, (23) |
| 434-449 | GPEVLGVDVFSEALAA, (16) |
| 450-463 | PLFWIAANAGLDGS, (14) |
| 464-471 | VVVNKVSE, (8) |
| 480-494 | VNTLSYGDLAADGVI, (15) |
| 501-526 | RSAVLNASSVARMVLTTETVVVDKPA, (15) |
| 526-539 | KAEDHDHHHGHAH, (14) |

7. The use as claimed in any previous claim wherein the condition to be treated is selected from at least one of the following conditions: allergic rhinitis, allergic asthma, intrinsic asthma, allergic bronchopulmonary aspergillosis, eosinophilic pneumonia, allergic bronchitis bronchiectasis, occupational asthma, reactive airway disease syndrome, or hypereosinophilic syndrome.

8. The use as claimed in Claim 7 wherein the condition is asthma.

9. The use as claimed in Claim 7 wherein the condition is allergic rhinitis.

10. The use as claimed in Claim 1 wherein the composition is an adjuvant.

11. The use as claimed in Claim 10 wherein the medicament further comprises an antigen.

12. The use as claimed in Claim 11 wherein the antigen is selected from at least one of the following: -
Anthrax, Cholera, Diphtheria, Haemophilus influenza b (Hib), Hepatitis A, Hepatitis B, Influenza, Japanese encephalitis, Measles, mumps and rubella (MMR), Meningococcal, Pertussis, Pneumococcal, Poliomyelitis, Rabies, Rubella, Smallpox and vaccinia, Tetanus, Tick borne encephalitis, Tuberculosis, Typhoid, Varicella/herpes zoster, Yellow fever and veterinary vaccine antigens.

13. The use as claimed in any one of Claims 1 to 12 wherein the medicament comprises a therapeutically or prophylactically effective dose, or plurality of doses, of the pharmaceutical composition.

14. The use of any previous claim wherein the medicament for the treatment and/or prevention of allergic airway diseases acts by stimulation of cytokine production in a diseased cell.

15. The use as claimed in Claim 14 wherein the cytokine production is stimulated at least 10-fold relative to normal levels.

16. The use as claimed in Claim 14 or 15 wherein the cytokines stimulated are selected from at least one of the group consisting of IL1β, IL-2, TL-6, IL-8, IL-10, IL-12, TNFα, interferon - y and GM-CSF.

## Patentansprüche

1. Verwendung einer pharmazeutischen Zusammensetzung bei der Herstellung eines Medikaments für die Prävention und/oder Behandlung von allergischen Luftwegs- und Atmungsorgan-Erkrankungen, die durch eine eosinophile Luftwegsentzündung und eine Luftwegs-Überempfindlichkeit **gekennzeichnet** sind, wobei die pharmazeutische Zusammensetzung ein Nukleinsäure-Molekül umfasst, das folgendes umfasst:
(i) die Nukleotidsequenz aus Fig. 1 oder
(ii) eine Sequenz, die zu mehr als 66 %, beispielsweise zu 70 % oder 75%, vorzugsweise zu mehr als 80%, z.B. mehr als 90% oder 95% Identität zur Sequenz (i) aufweist oder eine Sequenz, die unter Bedingungen von 2 x SSC, 65°C zur Sequenz (i) hybridisiert (wobei SCC = 0,15 M NaCl, 0,015 M Natriumzitrat und einen pH-Wert 7,2 bedeutet), die ein zu der durch die Nukleotidsequenz aus Fig. 1 kodierten Sequenz funktional äquivalentes Protein kodiert, oder
(iii) ein Fragment der Sequenz (i) oder (ii), das ein funktional äquivalentes Protein-Fragment kodiert, sowie einen pharmazeutisch akzeptablen Arzneimittelträger, Verdünner oder Träger.

2. Verwendung einer pharmazeutischen Zusammensetzung bei der Herstellung eines Medikaments für die Prävention und/oder Behandlung von allergischen Luftwegs- und Atmungsorgan-Erkrankungen, die durch eine eosinophile Luftwegs-Entzündung und durch eine Luftwegs-Überempfindlichkeit **gekennzeichnet** sind, wobei die pharmazeutische Zusammensetzung ein Polypeptid umfasst, das folgendes umfasst:
(i) die Aminosäure-Sequenz aus Fig. 1, oder
(ii) eine Sequenz, die zu mehr als 60%, beispielsweise 65% oder 70%, vorzugsweise mehr als 80%, beispielsweise mehr als 90% oder 95% zur Sequenz (i) homolog ist, die ein funktional äquivalentes Protein liefert, oder
(iii) ein funktional äquivalentes Fragment der Sequenz (i) oder (ii), und
einen pharmazeutisch akzeptablen Arzneimittelträger, Verdünner oder Träger.

3. Verwendung nach Anspruch 2, bei der die Fragmente eine Länge zwischen 6 und 400 Residuen besitzen.

4. Verwendung nach Anspruch 3, bei der die Fragmentlängen zwischen 6 bis 100 oder 15 bis 100 Residuen liegen.

5. Verwendung nach Anspruch 4, bei der die Fragmentlängen zwischen 6 bis 30, 10 bis 25, 15 bis 50 oder 15 bis 30 Residuen liegen.

6. Verwendung nach Anspruch 2, bei der die Fragmente von wenigstens einem der folgenden Residuen abgeleitet sind oder aus ihm bestehen:
| | |
|---|---|
| 1-8 | MSKLIEYD, (8) |
| 14-21 | AMEVGMDK, (8) |
| 40-48 | AKAFGGPTV, (9) |
| 64-71 | PFEDLGAQ, (8) |
| 96-105 | QALIKGGLRL, (11) |
| 110-129 | VNPIALGVGIGKAADAVSEA, (20) |
| 132-143 | ASATPVSGKTGI, (12) |
| 144-155 | AQVATVSSRDEQ, (12) |
| 160-175 | VGEAMSKVGHDGVVSV, (16) |
| 179-200 | STLGTELEFTEGIGFDKGFLSA, (22) |
| 195-219 | KGFLSAYFVTDFDNQQAVLEDALIL, (25) |
| 206-219 | FDNQQAVLEDALIL, (14) |
| 221-229 | HQDKISSLP, (9) |
| 264-271 | AIRKTLKA, (8) |
| 276-293 | GPYFGDRRKAFLEDLAVV, (18) |
| 299-314 | VNPDAGMVLREVGLEV, (16) |
| 315-326 | LGSARRVVVSKD, (12) |
| 327-342 | DTVIVDGGGTAEAVAN, (16) |
| 343-353 | RAKHLRAEIDK, (11) |
| 379-391 | VGAATETALKERK, (13) |
| 392-400 | ESVEDAVAA, (9) |
| 411-433 | PGGGASLIHQARKALTELRASLT, (23) |
| 434-449 | GPEVLGVDVFSEALAA, (16) |
| 450-463 | PLFWIAANAGLDGS, (14) |
| 464-471 | VVVNKVSE, (8) |
| 480-494 | VNTLSYGDLAADGVI, (15) |
| 501-526 | RSAVLNASSVARMVLTTETVVVDKPA, (15) |
| 526-539 | KAEDHDHHHGHAH, (14) |

7. Verwendung nach einem der vorhergehenden Ansprüche, bei welcher der zu behandelnde Zustand wenigstens einer der folgenden Zustände ist: allergische Rhinitis, allergisches Asthma, intrinsisches Asthma, allergische bronchopulmonare Aspergillose, eosinophile Pneumonie, allergische Bronchitis-Bronchiektase, berufsbedingtes Asthma, reaktives Luftwegs-Erkrankungs-Syndrom oder hypereosinophiles Syndrom.

8. Verwendung nach Anspruch 7, bei der der Zustand Asthma ist.

9. Verwendung nach Anspruch 7, bei der der Zustand allergische Rhinitis ist.

10. Verwendung nach Anspruch 1, bei der die Zusammensetzung ein Adjuvanz ist.

11. Verwendung nach Anspruch 10, bei der das Medikament weiterhin ein Antigen umfasst.

12. Verwendung nach Anspruch 11, bei der das Antigen wenigstens eines aus der folgenden Gruppe ist: Anthrax, Cholera, Diphtherie, Hämophilus influenzae Typ b (Hib), Hepatitis A, Hepatitis B, Influenza, japanische Encephalitis, Masern, Mumps und Röteln (MMR), Meningokokken, Keuchhusten, Pneumokokken, Kinderlähmung, Tollwut, Röteln, Windpocken, Vaccinia, Tetanus, durch Zecken übertragene Encephalitis, Tuberkulose, Typhus, Varicella/Herpes Zoster, Gelbfieber und Veterinär-Impf-Antigene.

13. Verwendung nach einem der Ansprüche 1 bis 12, bei der das Medikament eine therapeutisch oder prophylaktisch wirksame Dosis, oder eine Vielzahl von Dosen der pharmazeutischen Zusammensetzung enthält.

14. Verwendung nach einem der vorhergehenden Ansprüche, bei der das Medikament für die Behandlung und/oder Prävention von allergischen Luftwegs-Erkrankungen durch die Stimulation der Cytokin-Produktion in einer erkrankten Zelle wirkt.

15. Verwendung nach Anspruch 14, bei der die Cytokin-Produktion im Vergleich zu normalen Pegeln um wenigstens das 10-fache stimuliert wird.

16. Verwendung nach Anspruch 14 oder 15, bei der die stimulierten Cytokine wenigstens eines aus der folgenden Gruppe sind IL1β, IL-2, IL-6, IL-8, IL-10, IL-12, TNFα, Interferon-γ und GM-CSF.

## Revendications

1. Utilisation d'une composition pharmaceutique pour la préparation d'un médicament destiné à la prévention et/ou au traitement des maladies allergiques des voies aériennes et de maladies respiratoires **caractérisées par** une inflammation éosinophile des voies aériennes et une hypersensibilité des voies aériennes, dans laquelle la composition pharmaceutique comprend une molécule d'acide nucléique comprenant
(i) la séquence nucléotidique de la figure 1, ou
(ii) une séquence qui a plus de 66 %, par exemple 70 ou 75 %, de préférence plus de 80 %, par exemple plus de 90 ou 95 %, d'identité avec la séquence (i) ou une séquence qui s'hybride à la séquence (i) dans les conditions de 2 x SSC, 65 °C (où SSC = NaCl 0,15 M, citrate de sodium 0,015 M, pH 7,2) qui code une protéine fonctionnellement équivalente à la séquence codée par la séquence nucléotidique de la figure 1, ou
(iii) un fragment de la séquence (i) ou (ii) codant un fragment protéique fonctionnellement équivalent ; et un excipient, diluant ou véhicule pharmaceutiquement acceptable.

2. Utilisation d'une composition pharmaceutique pour la préparation d'un médicament destiné à la prévention et/ou au traitement de maladies allergiques des voies aériennes et de maladies respiratoires **caractérisées par** une inflammation éosinophile des voies aériennes et une hypersensibilité des voies aériennes, dans laquelle la composition pharmaceutique comprend un polypeptide comprenant
(i) la séquence d'acides aminés de la figure 1, ou
(ii) une séquence qui a plus de 60 %, par exemple 65 ou 70 %, de préférence plus de 80 %, par exemple plus de 90 ou 95 %, d'homologie avec la séquence (i) qui procure une protéine fonctionnellement équivalente, ou
(iii) un fragment de la séquence (i) ou (ii) fonctionnellement équivalent ; et
un excipient, diluant ou véhicule pharmaceutiquement acceptable.

3. Utilisation selon la revendication 2, dans laquelle les fragments sont longs d'entre 6 et 400 résidus.

4. Utilisation selon la revendication 3, dans laquelle les longueurs des fragments sont de 6 à 100 ou 15 à 100 résidus.

5. Utilisation selon la revendication 4, dans laquelle les longueurs des fragments sont de 6 à 30, 10 à 25, 15 à 50 ou 15 à 30 résidus.

6. Utilisation selon la revendication 2, dans laquelle les fragments sont issus de ou constitués par au moins l'un des résidus suivants :
| | |
|---|---|
| 1-8 | MSKLIEYD, (8) |
| 14-21 | AMEVGMDK, (8) |
| 40-48 | AKAFGGPTV, (9) |
| 64-71 | PFEDLGAQ, (8) |
| 96-105 | QALIKGGLRL, (11) |
| 110-129 | VNPIALGVGIGKAADAVSEA, (20) |
| 132-143 | ASATPVSGKTGI, (12) |
| 144-155 | AQVATVSSRDEQ, (12) |
| 160-175 | VGEAMSKVGHDGVVSV, (16) |
| 179-200 | STLGTELEFTEGIGFDKGFLSA, (22) |
| 195-219 | KGFLSAYFVTDFDNQQAVLEDALIL, (25) |
| 206-219 | FDNQQAVLEDALIL, (14) |
| 221-229 | HQDKISSLP, (9) |
| 264-271 | AIRKTLKA, (8) |
| 276-293 | GPYFGDRPKAFLEDLAVV, (18) |
| 299-314 | VNPDAGMVLREVGLEV, (16) |
| 315-326 | LGSARRVVVSKD, (12) |
| 327-342 | DTVTVDGGGTAEAVAN, (16) |
| 343-353 | RAKHLRAEIDK, (11) |
| 379-391 | VGAATETALKERK, (13) |
| 392-400 | ESVEDAVAA, (9) |
| 411-433 | PGGGASLIHQARKALTELRASLT, (23) |
| 434-449 | GPEVLGVDVFSEALAA, (16) |
| 450-463 | PLFWIAANAGLDGS, (14) |
| 464-471 | VVVNKVSE, (8) |
| 480-494 | VNTLSYGDLAADGVI, (15) |
| 501-526 | RSAVLNASSVARMVLTTETVVVDKPA, (15) |
| 526-539 | KAEDHDHHHGHAH, (14) |

7. Utilisation selon l'une quelconque des revendications précédentes, dans laquelle l'affection à traiter est choisie parmi au moins l'une des affections suivantes : rhinite allergique, asthme allergique, asthme non-allergique, aspergillose broncho-pulmonaire allergique, pneumonie éosinophile, bronchectasie bronchique allergique, asthme professionnel, syndrome d'affection respiratoire réactionnelle ou syndrome hyperéosinophile.

8. Utilisation selon la revendication 7, dans laquelle l'affection est l'asthme.

9. Utilisation selon la revendication 7, dans laquelle l'affection est une rhinite allergique.

10. Utilisation selon la revendication 1, dans laquelle la composition est un adjuvant.

11. Utilisation selon la revendication 10, dans laquelle le médicament comprend en outre un antigène.

12. Utilisation selon la revendication 11, dans laquelle l'antigène est choisi parmi au moins l'un des suivants :-
antigènes d'anthrax, de choléra, de diphtérie, de *Haemophilus influenza b* (Hib), d'hépatite A, d'hépatite B, d'influenza, d'encéphalite japonaise, de rougeole, d'oreillons et de rubéole (ROR), de méningococcie, de coqueluche, de pneumococcie, de poliomyélite, de rage, de rubéole, de variole et de vaccine, de tétanos, d'encéphalite à tiques, de tuberculose, de typhoïde, de varicelle/zona, de fièvre jaune et de vaccins vétérinaires.

13. Utilisation selon l'une quelconque des revendications 1 à 12, dans laquelle le médicament comprend une dose thérapeutiquement ou prophylactiquement efficace, ou une pluralité de doses, de la composition pharmaceutique.

14. Utilisation selon l'une quelconque des revendications précédentes, dans laquelle le médicament pour le traitement et/ou la prévention de maladies allergiques des voies aériennes agit par stimulation de la production de cytokine dans une cellule malade.

15. Utilisation selon la revendication 14, dans laquelle la production de cytokine est stimulée au moins 10 fois par rapport aux taux normaux.

16. Utilisation selon la revendication 14 ou 15, dans laquelle les cytokines stimulées sont choisies parmi au moins l'une du groupe constitué par IL-1β, IL-2, IL-6, IL-8, IL-10, IL-12, TNFα, interféron y et GM-CSF.
